# EUROPÄISCHE PATENTSCHRIFT

(11) **EP 1 723 936 B1**
(45) Veröffentlichungstag und Bekanntmachung des Hinweises auf die Patenterteilung: **18.03.2009**
(21) Anmeldenummer: 06009053.7
(22) Anmeldetag: 02.05.2006
(51) Int. Cl.: A61F 2/52, A41C 3/00, A41C 3/12

(54) **Brustprothese**
Breast prosthesis
Prothèse mammaire

(30) Priorität: 17.05.2005 DE 202005007677 U
(43) Veröffentlichungstag der Anmeldung: 22.11.2006
(73) Patentinhaber: AMOENA Medizin-Orthopädie-Technik GmbH, 83064 Raubling (DE)
(72) Erfinder: Stelter, Nils, 83101 Achenmühle (DE); Wild, Helmut, 83022 Rosenheim (DE)
(74) Vertreter: Laufhütte, Dieter

(56) Entgegenhaltungen:
- EP-A- 0 895 758
- DE-A1- 2 827 077
- DE-A1- 19 616 190
- DE-C1- 2 953 683
- DE-U1-3202004 007 32

## Beschreibung

Die Erfindung betrifft eine Brustprothese nach dem Oberbegriff des Anspruchs 1.

Beispielsweise aus der DE 27 01 627 A ist ein Verfahren zur Herstellung von Brustprothesen aus in Kunststofffolien eingeschweißten, der Brustform nachgebildeten schalenförmigen Körpern aus einer additionsvernetzenden ZweiKomponenten-Silikonkautschuk-Masse bekannt. Die mit diesem Verfahren hergestellten Prothesen sind in ihrem Aussehen und in ihrem Verhalten wegen der elastischen Weichheit, der Beweglichkeit, der Konsistenz sowie des Gewichts des verwendeten Materials der natürlichen Brust in nahezu idealer Weise nachgebildet. Die Brustprothesen werden möglichst unverrutschbar an der Brust der Trägerin befestigt. Hierzu ist es beispielsweise aus der EP 392 960 A bekannt, Brustprothesen der eingangs angegebenen Art innerhalb eines umlaufenden lippenförmigen Randes auf ihrer Rückseite mit einer umlaufenden, durch einen Absatz gebildeten Schulter zu versehen, auf der Haftstreifen oder Haftstücke befestigt sind, die mit den Haftbereichen von an den Körper der Frau durch hautfreundliche Klebemittel befestigte Streifen in der Weise zusammenwirken, dass die Prothese mit den auf der Haut klebenden Haltestreifen verbunden ist und von diesen wieder gelöst werden kann. Als Befestigungsmittel ist dabei beispielsweise eine Klettverbindung vorgesehen.

Zur Verbesserung des Tragkomforts der vorgenannten Brustprothesen ist es beispielsweise aus der DE 44 21 516 C1 bekannt geworden, eine Brustprothese mit textiler Rückseite zu schaffen, bei der ein Hohlraum zwischen der textilen Rückseite und der aus Kunststoff bestehenden Prothese mit Watte gefüllt werden kann.

Eine weitere Entwicklung ist aus der EP 1 232 733 B bekannt. Hier wird beschrieben, dass der Silikonkautschuk-Masse der Brustprothese ein sogenanntes PCM-Material beigemischt ist. Ein derartiges PCM-Material kann beispielsweise eine Paraffin-Substanz mit einem Schmelzbereich von ca. 33 °C bis 37 °C sein. Die als PCM-Material eingesetzten Paraffine sind speziell für wärmetechnische Anwendungen modifizierte Paraffine. Diese Wärmeparaffine weisen eine spezifische Schmelzwärme bzw. Schmelzenthalpie auf, die mit 130 kJ/kg bis 150 kJ/kg für organische Stoffe sehr hoch ist. Steigt die Hautoberflächentemperatur über dieses Temperaturniveau wird das PCM-Material aufgeschmolzen. Zum Aufschmelzen ist eine entsprechend hohe Schmelzenergie notwendig, die als Wärme von der Hautoberfläche abgeführt wird und dadurch zu einem angenehmen Kühleffekt beiträgt.

Es sind noch weitere Modifikationen von gattungsgemäßen Brustprothesen bekannt geworden, die teilweise darin bestehen, die Brustprothese als Mehrschichtprothese auszubilden, wobei der dem Körper nähere Teil der Brustprothese mit einem thixotropen Material gefüllt ist.

Aus der DE 196 16 190 A1 ist eine Brustprothese bekannt, bei der der weichelastische Kunststoffkörper eine der Form der natürlichen Brust nachgebildete Vorderseite aufweist, während die Rückseite einen großen Hohlraum umschließt, so dass diese Brustprothese nach dem Anlagen nur im Randbereich den Oberkörper der Trägerin berührt. Derartige Brustprothesen eignen sich dazu, die empfindliche Haut und das Narbengewebe bei einer frisch amputierten Trägerin zu schonen. Innerhalb des Randes des Kunststoffkörpers wird ein flächiges Teil angeordnet, das aus einem Gewebe oder Gewirk aus Mikrofasern bestehen kann. Zwischen diesem flächigen Teil und der Rückseite des Kunststoffkörpers ist ein großer Hohlraum gebildet, der insbesondere den natürlichen Eindruck der Brustprothese beim Tragen beeinträchtigt.

Aufgabe der Erfindung ist es, den natürlichen Eindruck der Brustprothese sowie den Tragekomfort beim Tragen der Brustprothese noch weiter zu verbessern.

Aufgabe der Erfindung ist es, den Tragekomfort beim Tragen einer Brustprothese noch weiter zu verbessern.

Erfindungsgemäß wird diese Aufgabe durch die Kombination der Merkmale des Anspruchs 1 gelöst. Der Kern der Erfindung besteht darin, dass zwischen der Brustprothese und der Fläche, auf der sie aufliegt, also in der Regel die Körperoberfläche der Trägerin, ein dreidimensionales Textil, das auch als sogenanntes "3-D-Textil" bezeichnet wird, angeordnet ist. Derartige 3-D-Textile sind üblicherweise Produkte, die dreilagig aufgebaut sind. Sie weisen eine Oberseite und eine Unterseite auf sowie eine zwischen diesen liegende Lage mit sogenannten "Abstandsfäden". Hierdurch ergibt sich ein ultraleichtes Material mit extrem hoher Luftverteilung, weicher Polstereigenschaften, sehr gutem Rückstellverhalten und guter Verformbarkeit. Bei Verwendung dieses 3-D-Textils auf der Rückseite der Prothese wird somit zunächst die Möglichkeit der Belüftung zwischen Prothese und Körper wesentlich erhöht, was sich auf den Tragekomfort entscheidend auswirkt. Zusätzlich ergibt sich ein angenehmes Tragegefühl durch die weichen Polstereigenschaften und die gute Verformbarkeit.

Besonders vorteilhafte Ausgestaltungen der Erfindung ergeben sich aus den sich an den Hauptanspruch anschließenden Unteransprüchen.

Demnach kann das 3-D-Textil schweißabsorbierende Fäden enthalten. Hierdurch kann der sich auf der Haut bildende Schweiß aufgesogen werden. Insbesondere wenn die 3-D-Textil-Zwischenlagen lösbar angeordnet sind, können sie regelmäßig gewechselt werden, was insbesondere nach entsprechender Schweißabsorbtion von Vorteil ist.

Das 3-D-Textil kann unmittelbar auf der Rückseite der Brustprothese wieder lösbar aufgebracht sein. Dabei kann die Lösbarkeit durch Velcro-Streifen, doppelseitiges Klebeband und/oder Druckknöpfe oder andere entsprechenden lösbaren Befestigungselemente erfolgen.

Die Brustprothese kann Aushöhlungen und Vertiefungen aufweisen, in welche Modularkissen einlegbar sind, die im Wesentlichen aus einer gefüllten Kammer bestehen. Grundsätzlich können in diesen Kissen Materialien wie beispielsweise ein PCM-Material oder aber auch im einfachsten Fall Wasser oder eine andere beliebige Flüssigkeit eingefüllt sein. Beim derartigen modularen Aufbau ist das 3-D-Textil unmittelbar auf der gefüllten Kammer lösbar bzw. nicht lösbar aufgebracht.

Eine weitere Lösung der Erfindung besteht darin, dass die Brustprothese in die Tasche eine Büstenhalters einlegbar ist, der auf seiner beim Tragen dem Körper zugewandten Seite ein 3-D-Textil aufweist. Der Schutz bezieht sich auch auf einen derartigen Büstenhalter, der dazu dient, entsprechende Brustprothesen aufzunehmen. Erfindungsgemäß hat dieser Büstenhalter ein oder zwei Taschen zur Aufnahme der Brustprothesen, wobei auf der beim Tragen dem Körper zugewandten Seite ein 3-D-Textil vorhanden ist.

Weitere Einzelheiten und Vorteile der Erfindung werden anhand eines in der Zeichnung dargestellten Ausführungsbeispiels näher erläutert. Es zeigen:
- Fig. 1:: den Aufbau einer Brustprothese nach einem ersten Ausführungsbeispiel der vorliegenden Erfindung,
- Fig. 2:: einen alternativen Aufbau gemäß einer zweiten Ausführungsform,
- Fig. 3 u. Fig. 4:: weitere alternative Aufbauten einer Brustprothese nach der vorliegenden Erfindung,
- Fig. 5:: einen Büstenhalter zur Aufnahme von Brustprothesen gemäß einer Ausgestaltung der vorliegenden Erfindung und
- Fig. 6:: einen Schnitt durch den Büstenhalter gemäß Fig. 5.

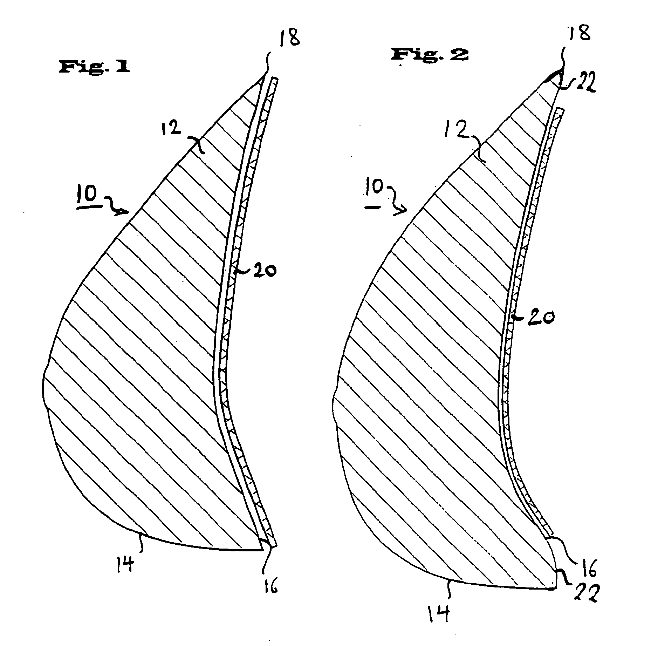

Die in Fig. 1 dargestellte Brustprothese 10 besteht aus einem schalenförmigen Körper 12, aus einer weich-elastisch eingestellten additionsvernetzenden Zweikomponenten-Kautschuk-Masse, dessen Außenseite durch eine Polyurethanfolie 14 und dessen Innenseite durch eine Polyurethanfolie 16 abdeckt ist, die längs eines gemeinsamen Umfangrandes 18 durch eine umlaufende Schweißnaht miteinander verbunden sind.

Auf der Rückseite der Brustprothese ist im Ausführungsbeispiel der Fig. 1 ein 3-D-Textil 20 angeordnet. Dieses 3-D-Textil deckt die Rückseite der Brustprothese 10 vollflächig ab.

Ein derartiges 3-D-Textil ist üblicherweise dreilagig aufgebaut und besteht aus einer Oberseite und einer Unterseite, zwischen denen sogenannte Abstandsfäden die Dicke des 3-D-Textils bestimmen. Die Dicke derartiger handelsüblicher 3-D-Textile wie sie in der vorliegenden erfindungsgemäßen Lösung angewandt werden, beträgt zwischen 1 mm bis über 20 mm. Typischerweise werden bei den 3-D-Textilien Polyester- oder auch Polyamidfasern verwendet. Es können aber auch speziell schweißabsorbierende Materialien in die 3-D-Textilien eingebaut sein.

Derartige 3-D-Textilien sind beispielsweise von der Firma Tytex unter dem Namen "AirX 3D Spacer Fabric", von der Firma Heathcoat unter dem Produktnamen "Spacetec", von der Firma Baltex unter der Bezeichnung "XD-Spacer Fabrics" oder von der Firma Müller-Textil unter der Bezeichnung "3mesh" bekannt. Durch die entsprechende Wahl der Dicke des 3-D-Textils kann die Luftzirkulation zwischen der Brustprothese 10 und dem hier nicht dargestellten Körper der Trägerin eingestellt werden. Darüber hinaus ergibt sich ein hoher Tragekomfort durch die weiche Polstereigenschaft und die gute Verformbarkeit des 3-D-Textils 20.

Die Fig. 2 entspricht im Wesentlichen der Fig. 1. Hier ist allerdings das 3-D-Textil derart angeordnet, dass jeweils ein Rand 22 übersteht. Entlang des Randes können Haftpads zum Befestigen der Brustprothese mit dem Körper der Trägerin verbunden sein.

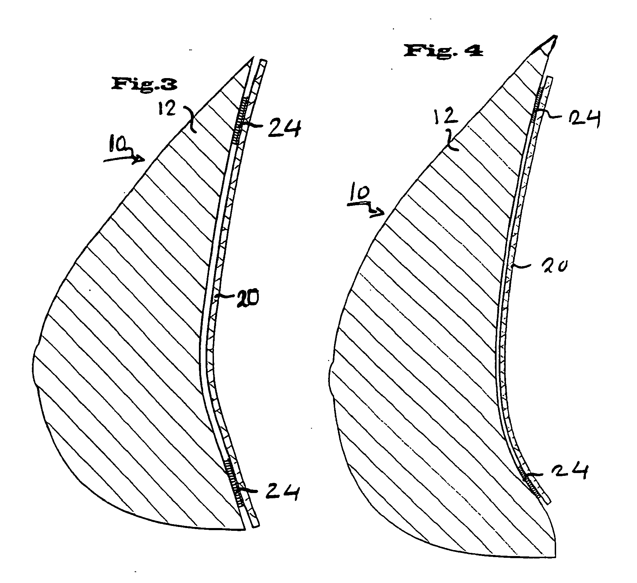

Die Figuren 3 und 4 entsprechen im Wesentlichen denjenigen gemäß Fig. 1 und 2. Hier ist jedoch jeweils ein Befestigungsmechanismus zum lösbaren Befestigen des 3-D-Textils 20 an der Brustprothese 10 gezeigt. Im hier dargestellten Ausführungsbeispiel handelt es sich um entsprechende Velcro-Bänder 24.

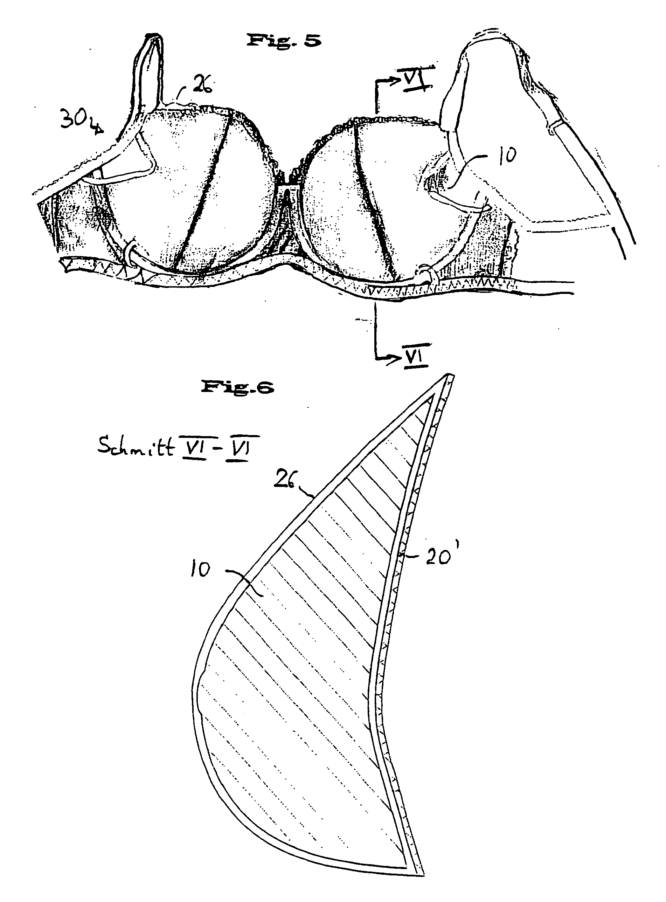

Eine andere Ausführungsvariante der Erfindung ergibt sich aus den Figuren 5 und 6. In Fig. 5 ist ein Büstenhalter 30 gezeigt, in welchem Taschen zur Aufnahme von Prothesen 10 ausgebildet sind. Die Schnittdarstellung gemäß der Schnittlinie VI-VI in Fig. 6 zeigt die textile Tasche 26 des Büstenhalters 30, in welchem die Brustprothese 10 eingelegt ist. Auf der Rückseite der Tasche 26 ist die Schicht 20' lösbar oder einteilig mit der Tasche 26 als 3-D-Textilschicht ausgebildet.

## Patentansprüche

1. Brustprothese (10) im wesentlichen bestehend aus in Kunststoffolien (14, 16) eingeschweißten, der Brustform nachgebildeten Körpern (12), vorzugsweise aus einer additionsvernetzenden Zweikomponenten-Siliconkautschuk-Masse, wobei zwischen der Brustprothese (10) und der Fläche, auf der sie aufliegt, ein dreidimensionales Textil, ein sogenamtes 3-D-Textil (20) angeordnet ist,
**dadurch gekennzeichnet,**
**dass** das dreidimensionale Textil (20) aus einer Oberseite und einer Unterseite, zwischen denen Abstandsfäden die Dicke des dreidimensionalen Textils bestimmen, besteht und dass das dreidimensionale Textil (20) unmittelbar auf der Rückseite der Brustprothese (10) aufgebracht ist.

2. Brustprothese nach Anspruch 1, **dadurch gekennzeichnet, dass** das 3-D-Textil (20) schweißabsorbierende Fasern enthält.

3. Brustprothese nach Anspruch 1 oder 2, **dadurch gekennzeichnet, dass** das 3-D-Textil (20) unmittelbar auf der Rückseite der Brustprothese (10) wieder lösbar aufgebracht ist.

4. Brustprothese nach Anspruch 3, **dadurch gekennzeichnet, dass** das 3-D-Textil (20) über Velcrostreifen, doppelseitigem Klebeband und/oder Druckknöpfe mit der Rückseite der Brustprothese (10) verbunden ist.

5. Brustprothese nach einem der Ansprüche 1 bis 4, **dadurch gekennzeichnet, dass** in die Brustprothese (10) Kissen, bestehend aus einer gefüllten Kammer, einlegbar sind.

6. Brustprothese nach einem der Ansprüche 1 bis 5, **dadurch gekennzeichnet, dass** zwischen dem 3-D-Texil (20) und dem Körper noch eine oder mehrere Lagen eines zusätzlichen Textils, beispielsweise Tüll oder ähnliches, angeordnet sind.

7. Büstenhalter mit einer Brustprothese nach einem der Ansprüche 1 bis 6, **dadurch gekennzeichnet, dass** die Brustprothese in eine Tasche des Büstenhalters einlegbar ist, der auf seiner beim Tragen dem Körper zugewandten Seite ein 3-D-Textil aufweist.

## Claims

1. Breast prosthesis (10) consisting essentially of cup-shaped bodies simulating the shape of the natural breast which are each sealed into films of plastic (14, 16) and preferably made from addition-cross linked two component silicone rubber material, wherein a three dimensional textile fabric, a so called a "3-D textile", is arranged between breast prosthesis (10) and the skin area on which it abuts,
**characerised in that**
the three dimensional textile fabric (20) consist of an upper face und a lower face and having spacer filaments sandwiched in between which define the thickness of the textile, and in that the three dimensional textile fabric (20) is applied adjacent to the rear face of the breast prosthesis (10).

2. Breast prosthesis according to claim 1, **characerised in that** the 3-D textile (20) contains fibers absorbing perspiration.

3. Breast prosthesis according to claim 1 or 2, **characerised in that** the 3-D textile (20) is detachably applied adjacent to the rear face of the breast prosthesis (10).

4. Breast prosthesis according to claim 3, **characerised in that** the 3-D textile (20) is attached to the rear face of the breast prosthesis (10) by Velcro strips, double sided tape and/or press buttons.

5. Breast prosthesis according to claim 1 to 4, **characerised in that** pads consisting of a filled chamber are inserted into the breast prosthesis (10).

6. Breast prosthesis according to claim 1 to 5, **characerised in that** between the 3-D textile (20) and the body of the woman wearing the prosthesis one ore more additional sheets of another fabric, for example tulle or something equivalent, are arranged.

7. Bra including breast prosthesis (10) according to one of the claims 1 to 6, **characerised in that** the breast prosthesis may be is inserted into a pocket of the bra having a 3-D textile on the face facing the woman's body while wearing the prosthesis.

## Revendications

1. Prothèse mammaire (10) consistant essentiellement en des corps (12) simulant la forme du sein naturel qui sont cachetés dans des feuilles en matière plastique (14, 16), consistant préférentiellement à une masse de caoutchouc silicone en deux composants reticulée par l'addition, un tissue textile tridimensionnel (20), dit textile 3-D etant arrangé entre le prothèse mammaire (10) et la surface de la peau à laquelle il aboutit,
characterisé par le fait que
le textile 3-D (20) consiste à une face supérieure et une face inférieure entre lesquelle des filaments d'espacement definissent l'épaisseur du textile tridimensionnel et que le textile tridimensionnel (20) est appliqué adjacent au revers du prothèse mammaire (10).

2. Prothèse mammaire (10) selon la revendication 1, characterisé par le fait que le textile 3-D (20) contient des filaments absorbant la transpiration.

3. Prothèse mammaire (10) selon la revendication 1 ou 2, characterisé par le fait que le textile 3-D (20) est mis adjacent au revers du prothèse mammaire (10) dans une manière détachable.

4. Prothèse mammaire (10) selon la revendication 3, characterisé par le fait que le textile 3-D (20) est lié au revers du prothèse mammaire (10) par des bandes Velcro, du ruban adhésif à double face et/ou des boutons-pression.

5. Prothèse mammaire (10) selon la revendication 1 à 4, characterisé par le fait que l'on peut insérer des coussinets consistant à une chambre remplie dans le prothèse mammaire (10).

6. Prothèse mammaire selon la revendication 1 à 5, characterisé par le fait que une ou plusieurs couches d'un tissue textile additional, par exemple du tulle ou d'autres choses semblables, sont situées entre le textile 3-D (20) et le corps de la porteuse.

7. Soutien-gorge avec un prothèse mammaire selon la revendication 1 à 6, characterisé par le fait que l'on peut insérer le prothèse mammaire dans une poche d'un soutien-gorge qui exhibit un textile 3-D sur la face tournée vers le corps de la femme en portant.
